# NEUE EUROPÄISCHE PATENTSCHRIFT

(11) **EP 0 605 497 B2**
(45) Veröffentlichungstag und Bekanntmachung des Hinweises auf die Entscheidung über den Einspruch: **14.04.1999**
(45) Hinweis auf die Patenterteilung: 20.03.1996
(21) Anmeldenummer: 92919477.7
(22) Anmeldetag: 16.09.1992
(51) Int. Cl.: A61K 9/16, A61K 9/51

(54) **ARZNEISTOFFTRÄGER AUS FESTEN LIPIDTEILCHEN (FESTE LIPIDNANOSPHÄREN (SLN))**
MEDICATION VEHICLES MADE OF SOLID LIPID PARTICLES (SOLID LIPID NANOSPHERES - SLN)
EXCIPIENT CONSTITUE DE PARTICULES LIPIDIQUES SOLIDES DITES NANOSPHERES LIPIDIQUES SOLIDES (SLN)

(30) Priorität: 18.09.1991 DE 4131562
(43) Veröffentlichungstag der Anmeldung: 13.07.1994
(73) Patentinhaber: MEDAC GESELLSCHAFT FÜR KLINISCHE SPEZIALPRÄPARATE GmbH, D-20354 Hamburg (DE)
(72) Erfinder: LUCKS, Stefan, D-2300 Kiel 1 (DE); MÜLLER, Rainer, D-2300 Kiel 1 (DE)
(74) Vertreter: UEXKÜLL & STOLBERG
(86) Internationale Anmeldenummer: EP9202132
(87) Internationale Veröffentlichungsnummer: WO9305768

(56) Entgegenhaltungen:
- EP-A- 0 167 825
- EP-A- 0 375 520
- EP-A- 0 418 153
- EP-A- 0 438 359
- EP-A- 0 506 197
- US-A- 4 533 254
- US-A- 4 880 634
- US-A- 4 908 154
- US-A- 5 039 527
- JOURNAL OF PHARMACEUTICAL SCIENCES Bd. 55, Nr. 4, April 1966, Seiten 376 - 380 DRAPER E.V. ET AL 'SOME WAX FORMULATIONS OF SULFAETHYLTHIADIAZOLE PRODUCED BY AQUEOUS DISPERSION FOR PROLONGED RELEASE MEDICATION'
- Handbook of Microfluidizer Applicatons, issued by Microfluidics Corporation
- C. Washington (1987), Laboratory Equipment Digest
- S.L. Silvestri et al (1989) Int. J. Pharm. 50, p. 141-146
- Pharmaceutical Dosage Forms : Disperse systems, Vol. 2 (ed H.A. Lieberman et al), Marcel Dekker, New York, 1989, p. 51-57
- Remington's Pharmaceutical Science (ed. A.R. Gennaro), Mack Publishing Company, 1990, p. 1545-1549, 1590
- H.P. Fiedler Lexikon des Hilfsstoffe für Pharmazie, Kosmetik und angrenzende Gebiete, 3rd Editio Cantor Aulendorf, 1989, p. 232
- H. Korstvedt et al (1985), American Paint and Coatings Journal, p. 38-39
- R.J. Prankerd et al (1988), J. Parent Sci. & Technol. 42(3), p. 76-81

## Beschreibung

Die Erfindung betrifft einen tensidfreien Arzneistoffträger, dessen Dispersion in einem wäßrigen Medium, ein Verfahren Zu seiner Herstellung und zur Herstellung tensidhaltiger Arzneistoffträger und seine Verwendung. Insbesondere handelt es sich um einen Arzneistoffträger aus Lipid- oder Lipoidteilchen.

Auf dem Gebiet der Arzneimittelwirkstoffe wird ständig nach Trägern gesucht, die eine vielfältige Art der Applikation ermöglichen, d.h. in einer Form vorliegen, die es gestattet, das jeweilige Medikament auf die am besten geeignete Weise dem Körper zuzuführen, z.B. intravenös, intraarthrikulär, intramuskulär oder subkutan.

So sind beispielsweise Träger aus festen Mikroteilchen, Mikrosphären und Mikrokapseln bekannt (mittlerer Durchmesser im Mikrometerbereich) sowie Nanoteilche und Nanokapseln (mittlerer Durchmesser im Nanometerbereich). Mikro- und Nanoteilchen bestehen aus einerfesten Polymermatrix. Bei Mikro- und Nanokapseln sind flüssige oder feste Phasen von filmbildenden Polymeren umhüllt. Derartige Teilchen bestehen aus oder weisen Überzüge aus Polymeren wie Polylactiden (PLA), Polylactid-Glycoliden (PLA/GA) oder Polyalkylcyanoacrylaten auf. Polylactid und Polylactid-Glycolid als Teilchenmatrix und als Überzüge haben jedoch den Nachteil, daß sie nur sehr langsam abgebaut werden, d.h. der Abbau dauert Wochen bis Monate. Dies führt bei Mehrfachapplikation eines Arzneimittels mit diesem Träger zur Polymerakkumulation im Organismus und möglicherweise zu toxischen Effekten. Teilchen auf Basis von Polymeren wie Polyalkylcyanoacrylaten werden zwar innerhalb von 24 Stunden bis zu 80% im Organismus abgebaut, doch wird beim Abbau toxisches Formaldehyd frei. Zur Herstellung der Polymerteilchen müssen als Lösungsmittel für das Polymer beispielsweise Chlorkohlenwasserstoffe wie Dichlormethan eingesetzt werden, die ihrerseits wiederum toxisch sind (T.R. Tice und D.H. Lewis, Microencapsulation Process, US-PS 4 389 330). Mikroteilchen können darüberhinaus aufgrund ihrer Größe bei der intravenösen Injektion zu Embolien führen, 50 daß hiervon in der Regel abgesehen wird. Ein weiterer Nachteil von Polymerteilchen ist, daß beim Sterilisieren in einem Autoklaven die Glastemperatur überschritten wird, so daß es zu einer Aggregation der Teilchen kommt. Derartige Arzneimittelträger bzw. Arzneimittel sind daher auf diese Weise nicht sterilisierbar und müssen auf dem mit Nachteilen behafteten Wege der Strahlensterilisation behandelt werden.

Bekannt sind ferner als Arzneistoffträger einsetzbare Fettemulsionen. Fettemulsionen sind Öl-in-Wasser-Emulsionen, bei denen die dispergierte (innere) Phase flüssig ist. In der Literatur werden derartige Fettemulsionen auch als ''Lipid-Mikrosphären'' und hochdisperse Fettemulsionen mit einer mittleren Teilchengröße im Nanometerbereich werden auch als "Nanoemulsionen" bezeichnet (H.G. Weder und M. Muetsch, Eur Pat. EP 90-810436, Juni 1990). Diese Fettemulsionen bestehen insgesamt aus zweiflüssigen Phasen. Fettemulsionen geben inkorporierte Arzneistoffe nach Verdünnung durch Körperflüssigkeiten (z.B. nach Injektion ins Blut) relativ rasch frei. Die t (50%) liegt im Bereich von 30 bis 60 Sekunden, was mit der hohen Diffusonsgeschwindigkeit der Arzneistoffe im relativ niedrigviskosen Öl korreliert ist. Zusätzlich wird dieflüssige dispergierte Phase der Fettemulsionen (= Öl) im Organismus innerhalb weniger Stunden vollständig metabolisiert, was zur schnellen Freisetzung auchvon extrem lipophilen Substanzen aus dem Öl führt. Durch die rasche Freisetzung kann es auch zu sogenannten Wirkstoffpeaks im Plasma kommen, so daß aufgrund dieser kurzzeitigen Überdosierung toxische Nebenwirkungen möglich sind. Darüber hinaus ist der Verlust an Wirkstoff vor Erreichung des Zielorgans beim passiven Targeting zu Leber- und Milzmakrophagen relativ groß.

Durch P. Eldem, P. Speiser und A. Hincal, Pharmaceutical Research **8**, 47-54 (1991) sind Mikropellets auf Lipidbasis bekannt, deren mittlerer Durchmesser wiederum im Miktometerbereich liegt.

Bekannt sind auch Arzneistoffräger, bei denen Liposomen oder Liposomen-ähnliche oder -analoge Substanzen wie Niosomen mit einem wäßrigen, flüssigen Kern von einer oder mehreren Phospholipiddoppelmembranen umgeben sind.

Darüber hinaus sind subpartikuläre oder halbpartikuläre Systeme bekannt, bei denen Substanzen mit Hilfe von Lösungsvermittlern wie Tensiden soweit gelöst werden, daß sich Mizellen oder Mischmizellen bilden. Hierbei handelt es sich nicht mehr um Dispersionen sondern bereits um Lösungen.

Aus der EP-A-0 167 825 ist ein arzneimittelhaltiges Trägersystem bekannt, daß aus Lipidnanopellets, die bei Raumtemperatur einen festen Aggregatzustand besitzen, mit einer Teilchengröße von 50 bis 1000 nm in Form einer wäßrigen, kolloidalen Suspension besteht, wobei die Lipidteilchen in der Suspeneon in einer Konzentration von 1 bis 20 Gew.% vorliegen, aus einem Gemisch von Lipiden mit grenzflächenaktiven Substanzen bestehen und 5 bis 70 Gew.% Lipide, 0,01 bis 70 Gew.% grenzflächenaktive Stoffe und 0,05 bis 25 Gew.% Wirkstoff enthalten. Das Einbringen der Wirkstoffe erfolgt direkt in das geschmolzene Lipid oder Lipidgemisch oder in ein Schmelzgemisch aus Lipid und grenzflächenaktivem Material oder wird durch Aufnehmen des grenzflächenaktiven Stoffen in einem organischen Lösungsmittel wie Chloroform und Einbringen dieser Lösung in das geschmolzene Lipid. Das Einmischen erfolgt durch Rühren mit einem handelsüblichen Rührwerk Schütteln oder Ultraschallbehandlung.

Der Erfindung liegt daher die Aufgabe zugrunde, einen tensidfreien Arzneistoffträger zur Verfügung zu stellen, der eine Dispersion von Teilchen in einem wäßrigen Medium bilden kann, wobei die Teilchen bei Raumtemperatur fest und biologisch abbaubar sind und außerdem aus Komponenten bestehen, die eine geringe oder keine Tozixität aufweisen. Bei der Herstellung des Arzneistoffträgers sollen ferner keine toxischen Hilfsstoffe wie halogenierte organische Lösungsmittel (Dichlormethan oder ähnliche) benötigt werden. Ferner soll ein Verfahren zur Herstellung diese Arzneistoffträgers oder eines entsprechenden tensidhaltigen Arzneistoffträgers zur Verfügung gestellt werden.

Die erfindungsgemäße Aufgabe wird gemäß Anspruch 1 durch ein Verfahren zur Herstellung eines Arzneistoffträgers gelöst, der tensidhaltige oder tensidfreie Teilchen aus Lipid, lipdähnlichem (lipoidem) Material oder Mischungen davon umfaßt, die einen Durchmesser von 10 nm bis 10 µm aufweisen, wobei die Teilchen der Hauptpopulation einen mittleren Durchmesser zwischen 40 und 1000 nm aufweisen und bei Raumtemperatur fest sind, wobei das Verfahren dadurch gekennzeichnet ist, daß entweder die innere Phase (das Lipid oder Lipoid) in geschmolzenem oder erweichtem Zustand in dem Dispersionsmittel (Wasser, wäßrige Lösung oder mit Wasser mischbare Flüssigkeit) hochdrucknomogenisiert wird oder die innere Phase in festem Zustand, wobei die feste Phase fein zerkleinert ist, in dem Dispersionsmittel hochdruckdispergiert wird.

Ferner wird sie durch einen Arzneistoffträger aus tensidfreien Teilchen gemäß Anspruch 21 gelöst.

Bevorzugte Ausgestaltungen dieses Arzneistoffträgers sind Gegenstand der Unteransprüche.

Bei dem nach dem erfindungsgemäßen Verfahren hergestellten Arzneistoffträger handelt es sich um bei Raumtemperatur (d.h. ca. 20°C) feste Teilchen mit einer Größe im Nanometerbereich. Derartige Teilchen können als "feste Lipidnanosphären'' (solid lipid nanospheres - SLN) bezeichnet werden. Diese Teilchen können in einem wäßrigen Medium dispergiert werden, so daß sich eine Fest/Flüssig-Dispersion ergibt. Die Teilchengröße der dispergierten Phase bewegt sich im Bereich von >10 nm bis zu wenigen Mikrometern (ca. 10 µm). Die mittlere Teilchengröße (Durchmesser bestimmt mit Photonenkorrelationsspektroskopie) liegt überwiegend im Bereich 100 bis 1000 nm, besonders 100 bis 800 nm. Durch geeignete Auswahl der Verfahrensparameter und durch geeignete Wahl von Hilfsstoffen (z.B. höhere Tensidkonzentration) ist es jedoch möglich, SLN kleiner als 100 nm, insbesondere im Bereich 40 bis 80 nm, herzustellen.

Die SLN bestehen aus Lipiden oder lipidähnlichen Substanzen, die vom Organismus wie Fett aus Nahrungsmitteln abgebaut werden können. Der Abbau von Lipiden erfolgt schneller als der Abbau von synthetischen Polymeren wie PLA, PLA/GA. Vorteilhafterweise entstehen ferner beim Abbau bzw. der Verstoffwechselung von Lipiden keine toxischen Metabolite wie es bei Teilchen auf Polyalkylcyanacrylatbasis der Fall ist. Diesbezüglich wird auf die Toxikologie der seit den 50er Jahren in der parenteralen Ernährung verwendeten Fettemulsionen verwiesen.

Da es sich bei den SLN um feste Lipidteilchen mit entsprechend hoher Viskosität handelt, ist die Diffusions- und Freisetzungsgeschwindigkeit eines darin eingeschlossenen Wirkstoffs reduziert. Somit ist es im Gegensatz zu Fettemulsionen mit flüssiger dispergierter Phase möglich, die Einstellung einer kontrollierten Freisetzung über einen längeren Zeitraum zu erreichen. Aufgrund der längeren Freisetzungszeit wird die Bildung von Plasmapeaks des jeweiligen Wirkstoffs vermieden, so daß die aufgrund derartiger Spitzenwerte eintretenden Nebenwirkungen aufbleiben. Ferner ist der Verlust an Wirkstoff nach Applikation und vor Erreichung des jeweiligen Zielorgans aufgrund der verzögerten Freisetzung geringer als bei Fettemulsionen, bei denen die Wirkstoffe vergleichsweise schnell freigesetzt werden.

Der oder die Wirkstoffe sind in den Lipid- oder Lipoidteilchen gelöst oder dispergiert. Ferner können sie an deren Oberfläche adsorbiert sein. Aufgrund des Feststoffcharakters können auch hydrophile Wirkstoffe in Form einer wäßrigen Wirkstofflösung in die Lipid- oder Lipoidphase eingearbeitet werden. Nach dieser Einarbeitung und der anschließenden Dispergierung der erhaltenen SLN in dem wäßrigen Dispersionsmedium entsteht ein System W/F/W, d.h. Wasser in Fett in Wasser. Der Lipidkern schließt hierbei die wäßrige Arzneistofflösung aufgrund seines festen Aggregatzustandes besser ein als es bei vergleichbaren multiplen Emulsionen Wasser in Öl in Wasser (W/Ö/W möglich ist.

Ein weiterer Vorteil der festen Lipidnanosphären ist, daß sie im Gegensatz zu Teilchen aus Polymer in einem Autoklaven sterilisierbar sind, ohne daß es zu einer Aggregation der Teilchen kommt. Auf diese Weise können die mit der Strahlensterilisation verbundenen Nachteile umgangen werden.

Im Gegensatz zu Miktoteilchen aus dem Mikrometerbereich sind die SLN aufgrund ihrer geringen Teilchengröße im Nanometerbereich auch problemlos ohne Gefahr der Embolie intravenös injizierbar.

Bei ihrer Herstellung müssen keine toxischen Hilfsstoffe wie z.B. leicht flüchtige Chlorkohlenwasserstofflösungsmittel eingesetzt werden.

Der erfindungsgemäße Arzneistoffträger kann auf folgende Weisen hergestellt werden:
1. Dispergieren der inneren Phase (des Lipids oder Lipoids) in geschmolzenem oder erweichtem Zustand. Die Dispergierung erfolgt oberhalb der Raumtemperatur und kann durch verschiedene, beispielsweise die unten beschriebenen Verfahren bewirkt werden.
2. Dispergieren der festen inneren Phase in festem Zustand. Die feste Phase wird hierfür fein zerkleinert und in Wasser oder in einem wäßrigen Medium dispergiert.

Der dispergierte, bei Raumtemperatur feste Lipid- oder Lipoidkern wurde zuvor mit einem oder mehreren Arzneistoffen beladen. Dies kann dadurch erfolgen, daß der Wirkstoff in dem Lipid/Lipoid gelöst oder dispergiert wird, an dessen Oberfläche adsorbiert wird oder in Form einer wäßrigen Lösung in dem Lipid/Lipoid dispergiert wird.

Als dispergierte Phase können Lipide und Lipoide im wertesten Sinne als Einzelverbindungen oder als Mischungen eingesetzt werden. Beispiele hierfür schließen natürliche und synthetische Triglyceride oder deren Mischungen, Monound Diglyceride alleine oder in Mischung untereinander oder mit z.B. Triglyceriden, natürliche und synthetische Wachse, Fettalkohole einschließlich ihrer Ester und Ether sowie Lipidpeptide ein. Insbesondere sind synthetische Mono-, Dir- und Triglycerde als Einzelsubstanzen oder in Mischung (z.B. Hartfett), Glycerintrifettsäureester (z.B. Glycerintrilaurat, -myristat, -palmitat, -stearat und -behenat) und Wachse wie z.B. Cetylpalmitat und Cera alba (gebleichtes Wachs, DAB 9) geeignet.

Der Anteil der inneren oder Lipidphase bezogen auf die Grundrezeptur beträgt 0,1 bis 30 Gew.% und insbesondere 1 bis 10 Gew.%.

Falls es zur Herstellung stabiler Dispersionen erforderlich sein sollte, dispersionsstabilisierende Zusätze zu verwenden, können diese zur Stabilisierung der Teilchen in Form von Reinsubstanzen oder in Form von Mischungen eingesetzt werden. Ihre vorhandene Menge kann bezogen auf das Gesamtgewicht der wäßrigen Dispersion im Bereich 0,01 bis 20 Gew.-% und vorzugsweise von 0,5 bis 5 Gew.-% liegen. Als stabilisierende Substanzen kommen in Frage:
a) Tenside, insbesondere ethoxylierte Sorbitanfettsäureester, Blockpolymere und Blockcopolymere (wie z.B. Poloxamere und Poloxamine), Polyglycerinether und -ester, Lecithine verschiedenen Ursprungs (z.B. Ei- oder Sojalecithin), chemisch modifizierte Lecithine (z.B. hydriertes Lecithin) als auch Phospholipide und Sphingolipide, Mischungen von Lecithinen mit Phospholipiden, Sterine (z.B. Cholesterin und Cholesterinderivate sowie Stigmasterin), Ester und Ether von Zuckern oder Zuckeralkoholen mit Fettsäuren oder Fettalkoholen (z.B. Saccharosemonostearat),
b) sterisch stabilisierende Substanzen wie Poloxamere und Poloxamine (Polyoxyethylen-Polyoxypropylen-Blockpolymere), ethoxylierte Sorbitanfettsäureester, ethoxylierte Mono- und Diglyceride, ethoxylierte Lipide und Lipoide, ethoxylierte Fettalkohole oder Fettsäuren und
c) Ladungsstabilisatoren bzw. Ladungsträger wie z.B. Dicetylphosphat, Phosphatidylglycerin sowie gesättigte und ungesättigte Fettsäuren, Natriumcholat, Natriumglykolcholat, Natriumtaurocholat oder deren Mischungen, Aminosäuren oder Peptisatoren wie Natriumcitrat (siehe J. S. Lucks, B.W. Müller, R.H. Müller, Int. J. Pharmaceutics **63**, 183-188 (1990)).
d) viskositätserhöhende Stoffe wie Celluloseether und -ester (z.B. Methylcellulose, Hydroxyethylcellulose, Hydroxypropylcellulose, Natriumcarboxymethylcellulose), Polyvinylderivate wie Polyvinylalkohol, Polyvinylpyrrolidon, Polyvinylacetat, Alginate, Polyacrylate (z B. Carbopol), Xanthane und Pektine.

Die Ladungsstabilisatoren werden gegegebenenfalls bezogen auf die Grundrezeptur vorzugsweise in einer Menge von 0,01 bis 10 % und besonders bevorzugt von 0,05 bis 2 % eingesetzt und die viskositatserhöhenden Stoffe werden gegebenenfalls bezogen auf die Grundrezeptur vorzugsweise in einer Menge von 0,01 bis 10%, bevorzugter von 0,1 bis 10% und besonders bevorzugt von 0,5 bis 5% eingesetzt.

Als äußere Phase (kontinuierliche Phase, Dispersionsmittel) werden Wasser, wäßrige Lösungen oder mit Wasser mischbare Flüssigkeiten wie Glycerin oder Polyethylenglykol verwendet. Die wäßrigen Lösungen können hierbei nichtisotonisch oder isotonisch sein. Als wäßrige Lösungen kommen Mischungen von Wasser mit einer oder mehreren anderen Komponenten wie beispielsweise Glycerin, Mannose, Glucose, Fructose, Xylose, Trehalose, Mannit, Sorbit, Xylit oder andere Polyole wie Polyethylenglykol sowie Elektrolyte wie Natriumchlorid in Frage. Diese Komponenten werden dann anteilig in der Grundrezeptur in einer Menge von 0,1 bis 50 % und bevorzugt 1 bis 30 % eingesetzt.

Die Herstellung der SLN erfolgt in der Regel durch Dispergieren der inneren Phase (des Lipids oder Lipoids), in der äußeren Phase (Wasser, wäßrige Lösung oder mit Wasser mischbare Flüssigkeit) oberhalb der Raumtemperatur (>20°C). Bei der Dispergierung wird vorteilhafterweise auf die Verwendung von Ultraschallstäben verzichtet, um eine Kontamination durch Metallpartikel (z.B. Ti) zu vermeiden. Die Temperatur wird so gewählt, daß sich die zu dispergierende Substanz im flüssigen Zustand befindet oder zumindest im erweichten Zustand vorliegt. Bei vielen Lipiden erfolgt die Dispergierung somit bei 70 bis 80°C. Die Herstellung erfolgt meist in zwei Schritten:
1. Herstellen einer Vordispersion, z.B. mit einem Rührer oder einem Rotor-Stator-Dispergierer (z.B. Ultra Turrax). Falls es erforderlich ist, erfolgt der Zusatz einer oder mehrerer disperaonsstabilisierender Substanzen.
2. Anschließende Dispergierung bei erhöhtem Druck in einem Hochdruckhomogenisator (z.B. ein Spalthomogenisator wie APV Gaulin oder French Press, ein Hochgeschwindigkeitshomogenisator wie der Mikrofluidizer). Bei gut dispergierbaren Systemen kann Schritt 1 entfallen.

Die Herstellung tensidfreier SLN erfolgt durch Dispersion der Lipid- oder Lipoidphase in einer wäßrigen Phase, die einen oder mehrere viskositätserhöhende Stoffe allein oder in Kombination mit anderen Stoffen wie Zuckern und Zukkeralkoholen, insbesondere Glucose, Mannose, Trehalose, Mannit, Sorbit sowie anderen enthält. Ferner kann eine Kombination des oder der viskositätserhöhenden Stoffe oder deren Kombination mit Zuckern oder Zuckeralkoholen darüberhinaus in weiterer Kombination mit Ladungsträgern verwendet werden. Beispiele für geeignete Ladungsträger sind: Natriumcitrat, Natriumpyrophosphat, Natriumsorbat.

Die Einarbeitung des oder der Wirkstoffe kann nach verschiedenen Methoden erfolgen. Beispielhaft seien genannt:
1. Lösen des Wirkstoffs in der inneren Phase.
2. Lösen des Wirkstoffs in einem mit der inneren Phase mischbaren Lösungsmittlel und Zugabe dieser Wrkstofflösung zur inneren Phase. Anschließend wird gegebenenfalls das Lösungsmittel teilweise oder vollständig entfernt.
3. Dispergieren des Wirkstoffs in der inneren Phase (z.B. durch Dispergieren eines Feststoffs oder gezielte Präzipitation).
4. Lösen des Wirkstoffs in der aüßeren, wäßrigen Phase (z.B. amphiphile Substanzen) und Einbindung des Wrkstoffs in einen die Teilchen stabilisierenden Tensidfilm während der Herstellung.
5. Adsorption des Wirkstoffs an der Teilchenoberfläche.
6. Lösen des Wirkstoffs in der Lipid-/lipoiden Phase mittels eines Lösungsvermittlers (z.B. eines Blockcopolymeren oder Sorbitanfettsäureesters), anschließende Dispergierung der Lipid-/lipoiden Phase zur Herstellung der Vordispersion. Der Wirkstoff liegt dann in den SLN als feste Lösung vor.
7. Einarbeiten von wäßrigen Wirkstofflösungen in die Lipid-/lipoide Phase und anschließende Dispergierung der Lipid-/lipoiden Phase zur Herstellung der Vordispersion, so daß ein System W/F/W entsteht, das den multiplen Emulsionen analog ist.

Die Sterilisierung kann nach Verfahren erfolgen, die in den Arzneibüchern beschrieben sind, z.B. durch Autoklavieren (121°C, 2 bar, DAB 9) oder nach sonstigen anerkannten Verfahren.

Die Anwendungsgebiete für den erfindungsgemäßen Arzneistoffträger mit den festen Lipidnanosphären sind vielfältig. Beispielsweise kann er zur parenteralen, enteralen, pulmonalen und topischen (nasal, dermal, intraocculär) und in Körperhöhlen Arzneistoffapplikationen verwendet werden.

Bei der parenteralen Applikation handelt es sich insbesondere um:
1. Intravenöse Gabe (Targeting zu Leber, Milz und Knochenmark im Blut zirkulierenden Teilchen mit kontrollierter Freisetzung von Wirkstoffen, z.B. Peptidarzneistoffe, Cytostatika, Immunstimulantien, Wachstumsfaktoren wie der Colony Stimulating Factor (Leucozytenregulation) und der Growth Factor.
2. Intramuskuläre Gabe (Depotformen für verlängerte oder langanhaltende Abgabe von Wirkstoffen, z.B. Peptidarzneistoffen oder Hormonen).
3. Intraarthrikuläre Gabe (z.B. für Antirheumatika und Imunsuppressiva bei Arthritis).
4. Intrakavitale Gabe (z.B. für Cytostatika und Peptidarzneistoffe für Krebsformen im Peritoneum und in der Pleurahöhle) und
5. Subkutane Gabe (z.B. Depotformen für Cytostatika bei Hautkrebs).

Die enteralen Applikationsformen dienen insbesondere zur
1. Einarbeitung von lipidlöslichen Vitaminen,
2. lymphatischen Adsorption (z.B. Wirkstoff-Targeting von Cytostatika zu den Lymphknoten),
3. Präsentation von Antigenen (z.B. orale Immunisierung mit Hilfe der Peyerschen Plaques) und
4. Aufnahme von Peptidarzneistoffen mit Hilfe von M-Zellen.

Als pulmonale Applikationsformen kommen insbesondere in Betracht:
1. Aerosole, Dosieraerosole (Versprühen der wäßrigen SLN-Dispersion),
2. Instillation der Dispersion.

Als topische Anwendung seien beispeilhaft
1. dermatologische Arzneimittel zur Applikation von z.B. Cortikoiden und Antimykotika,
2. Augentropfen oder Augengele, z.B. für ß-Blocker, aber auch
3. Kosmetika analog den liposomalen Präparaten genannt.

### Beispiele für in SLN einzuarbeitende Arzneistoffe (als Salz, Ester, Ether oder in freier Form)

### Analgetika/Antirheumatika

Morphin, Codein, Piritamid, Fentanyl und Fentanylderivate, Leyomethadon, Tramadol, Diclofenac, Ibuprofen, Indometacin, Naproxen, Piroxicam, Penicillamin

### Antiallergika

Pheniramin, Dimetinden, Terfenadin, Astemizol, Loratidin, Doxylamin, Meclozin, Bamipin, Clemastin

### Antibiotika/Chemctherapeutika

hiervon: Polypeptidantibictika wie Colistin, Polymyxin B, Teicplanin, Vancomycin; Malariamittel wie Chinin, Halofantrin, Mefloquin, Chloroquin, Virustatika wie Ganciclovir, Foscarnet, Zidovudin, Aciclovir und andere wie Dapson, Fosfomycin, Fusafungin, Trimetoprim

### Antiepileptika

Phenytoin, Mesuximid, Ethosuximid, Primidon, Phenobarbital, Valproinsäure, Carbamazepin, Clonazepam

### Antimykotika

a) intern:
   Nystatin, Natamycin, Amphotericin B, Flucytoan, Miconazol, Fluconazol, Itraconazol
b) extern außerdem:
   Clotrimazol, Econazol, Tioconazol, Fenticonazol, Bifonazol, Oxiconazol, Ketoconazol, Isoconazol, Tolnattat

### Corticoide (Interna)

Aldosteron, Fludrocortison, Betametason, Dexametason, Triamcinolon, Fluocortolon, Hydroxycortison, Prednisolon, Prednyliden, Cloprednol, Methylprednisolon

### Dermatika

a) Antibiotika:
   Tetracyclin, Erythromycin, Neomycin, Gentamycin, Clindamycin, Framycetin, Tyrothricin, Chlortetracyclin, Mipirocin, Fusidnsäure
b) Virustatika wie oben, außerdem:
   Podophyllotoxin, Vidarabin, Tromantadin
c) Corticoide wie oben, außerdem:
   Amcinonid, Flupredniden, Alclometason, Clobetasol, Diflorason, Halcinonid, Fluocinolon, Clocortolon, Flumetason, Diflucortolon, Fludroxycortid, Halometason, Desoximetason, Fluocinolid, Fluocortinbutyl, Flupredniden, Prednicarbat, Desonid

### Diagnostika

a) radioaktive Isotope wie Te99m, In111 oder I131, kovalent gebunden an Lipide oder Lipoide oder andere Moleküle oder in Komplexen
b) hochsubstituiertre iodhaltige Verbindungen wie z.B. Lipide

### Hämostyptika/Antihämorrhagika

Blutgerinnungsfaktoren VIII, IX

### Hypnotika, Sedativa

Cyclobarbital, Pentobarbital, Phenobarbital, Methaqualon, Benzodiazepine (Flurazepam, Midazolam, Nitrazepam, Lormetazepam, Flunitrazepam, Triazolam, Brotizolam, Temazepam, Loprazolam)

### Hypophysen-, Hypothalamushormone, regulatorische Peptide und ihre Hemmstoffe

Corticotrophin, Tetracosactid, Choriongonadotropin, Urofollitropin, Urogonadotropin, Somatropin, Metergolin, Bromocriptin, Terlipressin, Desmopressin, Oxrtocin, Argipressin, Ornipressin, Leuprorelin, Triptorelin, Gonadorelin, Buserelin, Nafarelin, Goselerin, Somatostatin

### Immuntherapeutika und Zytokine

Dimepranol-4-acetatamidobenzoat, Thymopentin, α-Interferon, β-Interferon, γ-Interferon, Filgrastim, Interleukine, Azathioprin, Ciclosporin

### Lokalanaesthetika

intern:
   Butanilicain, Mepivacain, Bupivacain, Etidocain, Lidocain, Articain, Prilocain,
extern außerdem:
   Propipocain, Oxybuprocain, Tetracain, Benzocain

### Migränemittel

Proxibarbal, Lisurid, Methysergid, Dihydroergotamin, Clonidin, Ergotamin, Pizotifen

### Narkosemittel

Methohexital, Propofol, Etomidat, Ketamin, Alfentanil, Thiopental, Droperidol, Fentanyl

### Nebenschilddrüsenhormone, Calciumstoffwechselregulatoren

Dihydrotachysterol, Calcitonin, Clodronsäure, Etidronsäure

### Opthalmika

Atropin, Cyclodrin, Cyclopentolat, Homatropin, Tronicamid, Scopolamin, Pholedrin, Edoxudin, Idouridin, Tromantadin, Aciclovir, Acetazolamid, Diclofenamid, Carteolol, Timolol, Metipranolol, Betaxolol, Pindolol, Befunolol, Bupranolol, Levobununol, Carbachol, Pilocarpin, Clonidin, Neostigmin

### Psychopharmaka

Benzodiazepne (Lorazepam, Diazepam), Clomethiazol

### Schilddrüsentherapeutika

1-Thyroxin, Carbirnazol, Thiamazol, Propylthiouracil

### Sera, Immunglobuline, Impfstoffe

a) Immunglobuline allgemein und spezifisch wie Hepatitis-Typen, Röteln, Cytomegalie, Tollwut, FSME, VaricellaZoster, Tetanus, Rhesusfaktoren
b) Immunsera wie Botulismus-Antitoxin, Diphterie, Gasbrand, Schlangengift, Skorpiongift
c) Impfstoffe wie Influenza, Tuberkulose, Cholera, Diphterie, Hepatitis-Typen, FSME, Röteln, Hämophilus influenzae, Masern, Neisseria, Mumps, Poliomyelitis, Tetanus, Tollwut, Typhus

### Sexualhormone und ihre Hemmstoffe

Anabolika, Androgene, Antiandrogene, Gestagene, Estrogene, Antiestrogene (Tamoxifen etc.)

### Zystostatika und Metastasenhemmer

a) Alkylantien wie Nimustin, Melphalan, Carmustin, Lomustin, Cyclcphosphamid, Ifosfamid, Trofosfamid, Chlorambucil, Busulfan, Treosulfan, Prednimustin, Thiotepa
b) Antimetabolite wie Cytarabin, Fluorouracil, Methotrexat, Mercaptopurin, Tioguanin
c) Alkaloide wie Vinblastin, Vincristin, Vindesin
d) Antibiotika wie Aclarubicin, Bleomycin, Dactinomycin, Daunorubicin, Doxorubicin, Epirubicin, Idarubicin, Mitomycin, Plicamycin
e) Komplexe von Nebengruppenelementen (z.B. Ti, Zr, V, Nb, Ta, Mo, W, Pt) wie Carboplatin, Cisplatin und Metallocenverbindungen wie Titanocendichlorid
f) Amsacrin, Dacarbazin, Estramustin, Etoposid, Hydroxycarbamid, Mitoxanthron, Procarbazin, Temiposid
g) Alkylamidophospholipide (beschrieben in J.M. Zeidler, F. Emling, W. Zimmermann und H.J. Roth, Archiv der Pharmazie, 324 (1991), 687)
h) Etherlipide wie Hexadecylphosphocholin, Ilmofosin und Analoga, beschrieben in R. Zeisig, D. Arndt und H. Brachwitz, Pharmazie 45 (1990), 809-818.

Die Erfindung wird in den folgenden Beispielen näher erläutert.

### Beispiel 1

- 10,0 g: Cera Alba (gebleichtes Wachs)
- 2,5 g: Poloxamer 188 (Polyoxyethylen-Polyoxypropylen-Blockpolymer)
- 0,1 g: Dicetylphosphat
- 87,4 g: Wasser für Injektionszwecke

Gera alba und Dicetylphosphat wurden auf 70° erwärmt und mit der ebenfalls auf 70°C erwärmten Lösung von Poloxamer 188 in Wasser für Injektionszwecke gemischt. Die Mischung wurde mit Hilfe eines Ultra Turrax bei 70°C vordispergiert. Die so erhaltene Vordispersion wurde anschließend durch einen auf 70°C temperierten APV Gaulin Hochdrucknomogenisator gegeben (5 Zyklen mit 500 bar). Es wurde eine SLN-Dispersion mit einem mittleren Durchmesser von 216 nm erhalten. Der Polydispersrtätsindex als Maß für die Breite der Teilchengrößenverteilung betrug 0,143 (PCS-Photonenkorrelationsspektroskopie). Alle Partikel waren kleiner als 6,0 µm (vermessen mit einem Sympatek Laserdiffraktometer).

### Beispiel 2:

- 10,0 g: Cetylpalmitat
- 2,5 g: Poloxamer 188
- 87,5 g: Wasser für Injektionszwecke

Die Herstellung erfolgte wie unter Beispiel 1 beschrieben. Der mittlere Durchmesser betrug 215 nm, der Polydispersitätsindex 0,131 (PCS-Daten). Alle Partikel waren kleiner als 4,2 µm (Laserdiffraktometer).

### Beispiel 3:

- 10,0 g: Cetylpalmitat
- 2,5 g: Lipoid S 75 (Sojalecithin mit 75% Phosphatdylcholin)
- 0,1 g: Dicetylphosphat
- 87,4 g: Wasser für Injektionszwecke

Die Herstellung erfolgte wie unter Beispiel 1 beschrieben, jedoch wurde Lipoid S 75 in der erwärmten Lipidphase dispergiert. Der mittlere Durchmesser betrug 183 nm, der Polydispersitätsindex 0,133 (PCS-Daten). Alle Partikel waren kleiner als 8,6 µm (Laserdiffraktometer.

### Beispiel 4:

- 10,5 g: Glycerintrilaurat (Dynasan®112)
- 2,5 g: Poloxamer 188
- 87,5 g: Wasser für Injektionszuwecke

Die Herstellung erfolgte wie unter Beispiel 1 beschrieben. Der mittlere Durchmesser betrug 199 nm, der Polydispersitätsindex 0,180 (PCS-Daten). Alle Partikel waren kleiner als 7,2 µm (Laserdffiraktometer).

### Beispiel 5:

- 10,0 g: Cetylpalmitat
- 2,5 g: Poloxamer 188
- 0,5 g: Dicetylphosphat
- 87,0 g: Wasser für Injektiorszwecke

Die Herstellung erfolgte wie unter Beispiel 1 beschrieben. Die Kenndaten vor und nach der Autoklavierung belegen die Anwendbarkeit der Sterilisationsmethode;

| | mittlerer Durchmesser | Polydispersitätsindex | alle Partikel kleiner als |
|---|---|---|---|
| vor Sterilisation | 215 nm | 0,131 | 4,2 µm |
| nach Sterilisation | 214 nm | 0,109 | 3,0 µm |

### Beispiel 6:

Als Modellarzneistoff wurden 0,25 g Tetracainbase in die Rezeptur Nr. 5 eingearbeitet.

Die Herstellung erfolgte wie unter Beispiel 1 beschrieben. Der mittlere Durchmesser betrug 218 nm, der Polydispersitätsindex 0,186 (PCS-Daten). Alle Partikel waren kleiner als 10,2 µm (Laserdiffraktometer). Die Arzneistoffbeladung betrugt 92,8%

### Beispiel 7:

Als Modellarzneistoff wurde Tetracainbase in folgende Rezeptur eingearbertet:
- 10,0 g: Glycerintrilaurat (Dynasan 112)
- 5,0 g: Lipoid S 75 Tetracainbase 0,1 g, 0,5 g, 1,0 g oder 2,0 g
- auf 100,0 g: Wasser für Injektionszwecke

Die Herstellung erfolgte wie unter Beispiel 1 beschrieben, jedoch erfolgte die Hochdruckhomogenisation bei 1500 bar (drei Zyklen). Als mittlere Durchmesser (PCS-Daten) wurden folgende Werte erhalten:

| Arzneistoffgehalt (bezogen auf Lipidphase) | PCS-Durchmesser (nm) |
|---|---|
| 1 % | 103 |
| 5 % | 102 |
| 10 % | 101 |
| 20 % | 125 |

### Beispiel 8:

Die unter Beispiel 7 genannten Präparationen wurden gemäß DAB 9 autoklaviert (A 121).

| Arzneistoffgehalt (bez. auf Lipidphase) | PCS-Durchmesser vor Autoklavieren | PCS-Durchmesser nach Autoklavieren |
|---|---|---|
| 1 % | 103 nm | 101 nm |
| 5 % | 102 nm | 102 nm |
| 10 % | 101 nm | 95 nm |

Die Teilchen können ferner bei Verwendung hydrolyseempfindlicher Wirkstoffe lyophilisiert oder sprühgetrocknet werden.

### Beispiel 9:

- 10.0 g: Glycerintrilaurat (Dynasan 112)
- 5,0 G: Lipoid S 75
- 0,5 g: Tetracainbase
- 84,5 ml: wäßrige Glucoselösung (30% m/V)

Die Herstellung erfolgte wie unter Beispiel 1 beschrieben, jedoch erfolgte die Hochdruckhomogenisation bei 1500 x 10⁵ Pa (1500 bar) (drei Zyklen). Als mittlere Durchmesser (PCS-Daten) wurden folgende Werte vor und nach Lyophylisation erhalten:

| | Mittlerer Durchmesser | Polydispersitätsindex |
|---|---|---|
| vor Lyophilisation | 90 nm | 0,277 |
| nach Lyophilisation | 481 nm | 0,289 |

Als werterer Arzneistoff wurde der Wirkstoff Hexadecylphosphocholin (HPC) in eine Modellrezeptur eingearbeitet.

### Beispiel 10:

- 10,0 g: Glycerintrilaurat (Dynasan 112)
- 5,0 g: Poloxamer 188
- 0,1 g: Hexadecylphosphocholin
- 84,9 g: Wasser für Injektionszwecke

Die Herstellung erfolgte wie in Beispiel 1 beschrieben. Die erhaltene SLN-Dispersion hatte einen mittleren PCS Durchmesser von 178 nm. Der Polydispersitätsindex betrug 0,1653. Alle Partikel waren kleiner als 3,6 µm (Laserdiffraktometer). Durch Variation des Tensidgehalts oder der Tensidkomponente war es möglich, SLN-Dispersionen mit einem HPC-Gehalt von 0,1 bis 50 mg/g herzustellen.

Unter Verwendung viskositätserhöhender Stoffe ist es möglich, tensidfreie SLN-Dispersionen herzustellen.

### Beispiel 11:

- 10,0 g: Glycerintrilaurat (Dynasan 112)
- 0,5 g: Tylose MH 300
- 89,5 g: Wasser für Injektionszwecke

Die Herstellung erfolgte wie unter Beispiel 1 beschrieben, jedoch erfolgte die Hochdrucknomogenisation bei 500 x 10⁵ Pa (500 bar) (drei Zyklen). Der PCS-Durchmesser der Hauptpopulation betrug 879 nm mit einem Polydispersitätsindex von 0,367.

Durch Variationen der Verfahrensbedingungen ist es möglich, SLN-Dispersionen mit einem mittleren PCS-Durchmesser unter 100 nm herzustellen.

### Beispiel 12:

- 10,0 g: Glycerintrilaurat (Dynasan 112)
- 5,0 g: Lipoid S 75
- 85,0 g: Wasser für Injektionszwecke

Die Herstellung erfolgte wie unter Beispiel 1 beschrieben, jedoch erfolgte die Hochdruckhomogenisation bei 1500 x 10⁵ Pa (1550 bar) (drei Zyklen).

Der PCS-Durchmesser der Hauptpopulation betrug 88 nm (erhalten durch Polydispersrtätsanalyse mittels Fouriertransformation der erhaltenen Korrelationsfunktion).

Die Erfindung umfaßt auch das Verfahren zur Herstellung des beschriebenen Arzneimittelträgers sowie dessen Verwendung zur Applikation von Arzneimittelwirkstoffen.

Insgesamt gesehen, kombinieren die festen Lipidnanosphären die Vorteile von Polymernanopartikeln (fester Kern, kontrollierbare Freisetzung über einen längeren Zeitraum, Einarbeitungsmöglichkeit für hydrophile Arzneistoffe) mit den Vorteilen von parenteralen Fettemulsionen (relativ schnelle Abbaubarkert, geringe bzw. keine Toxizität, Herstellung im industriellen Maßstab mit bei der Emulsionsproduktion etablierten Techniken, problemlose Sterilisation durch Autoldavieren) unter Umgehung der Nachteile von Nanopartikeln (zu langsamer Abbau in vivo bzw. toxische Abbauprodukte, fehlende Scaling-up-Möglichkeit in der Produktion) und der Nachteile von Fettemulsionen (z. B. sehr schnelle Metabolisierung, sehr schnelle Arzneistofffreisetzung).

## Patentansprüche (Patentansprüche für folgende(n) Vertragsstaat(en): AT, BE, CH, DE, DK, ES, FR, GB, GR, IT, LI, LU, MC, NL, SE)

1. Verfahren zur Herstellung eines Arzneistoffträger, der tensidhaltige oder tensidfreie Teilchen aus Lipid, lipidähnlichem (lipoidem) Material oder Mischungen davon umfaßt, die einen Durchmesser von 10 nm bis 10 µm aufweisen, wobei die Teilchen der Hauptpopulation einen mittleren Durchmesser zwischen 40 und 1000 nm aufweisen und bei Raumtemperatur fest sind, dadurch gekennzeichnet, daß entweder die innere Phase (das Lipid oder Lipoid) in geschmolzenem oder erweichtem Zustand in dem Dispersionsmittel (Wasser, wäßrige Lösung oder mit Wasser mischbare Flüssigkeit) hochdruckhomogenisiert wird, wobei bei Verwendung von geschmolzenem Lipid oder Lipoid als innerer Phase während der Hochdruckhomogeni` sation auch ein oder mehrere Wirkstoffe zugegen ist/sind und in dem Lipid oder Lipoid einschlossen wird/werden, oder die innere Phase in festem Zustand, wobei die feste Phase fein zerkleinert ist, in dem Dispersionsmittel hochdruckdispergiert wird.

2. Verfahren zur Herstellung eines Arzneistoffträger, der tensidhaltige oder tensidfreie Teilchen aus Lipid, lipidähnlichem (lipoidem) Material oder Mischungen davon umfaßt, die einen Durchmesser von 10 nm bis 10 µm aufweisen, wobei die Teilchen der Hauptpopulation einen mittleren Durchmesser zwischen 40 und 1000 nm aufweisen und bei Raumtemperatur fest sind, dadurch gekennzeichnet, daß entweder die innere Phase (das Lipid oder Lipoid) in geschmolzenem oder erweichtem Zustand in dem Dispersionsmittel (Wasser, wäßrige Lösung oder mit Wasser mischbare Flüssigkeit) hochdruckhomogenisiert wird, oder die innere Phase in festem Zustand, wobei die feste Phase fein zerkleinert ist, in dem Dispersionsmittel hochdruckdispergiert wird und der Arzneistoffträger einen oder meherer Wirkstoffe umfaßt, der/die ausgewählt ist/sind aus:
Analgetika/Antirheumatika ausgewählt aus
Morphin, Codein, Piritamid, Fentanyl und Fentanylderivaten, Levomethadon, Tramadol, Diclofenac, Ibuprofen, Naproxen, Piroxicam, Penicillamin;
Antiallergika;
Antibiotika/Chemotherapeutika ausgewählt aus
Polypeptidantibiotika; Malariamitteln, den Virustatika Ganciclovir, Foscarnet, Zidovudin, Aciclovir und Dapson, Fosfomycin, Fusafungin, Trimetoprim;
Antiepileptika;
Antimykotika ausgewählt aus
Nystatin, Natamycin, Amphotericin B, Flucytosin, Miconazol, Fluconazol, Itraconazol, Clotrimazol, Econazol, Tioconazol, Fenticonazol, Bifonazol, Ketoconazol, Isoconazol, Tolnaftat;
Corticoiden ausgewählt aus
Aldosteron, Fludrocortison, Betametason, Dexametason, Fluocortolon, Prednisolon, Prednyliden, Cloprednol, Methylpredinsolon;
Dermatika ausgewählt aus
a) den Antibiotika Tetracyclin, Erythromycin, Neomycin, Gentamycin, Clindamycin, Framycetin, Tyrothricin, Chlortetracyclin, Mipirocin, Fusidinsäure
b) den Virustatika Podophyllotoxin, Vidarabin, Tromantadin,
c) den Corticoiden Amcinonid, Flupredniden, Alclometason, Clobetasol, Diflorason, Halcinonid, Fluocinolon, Clocortolon, Flumetason, Diflucortolon, Fludroxycortid, Halometason, Desoximetason, Fluocinolid, Fluocortinbutyl, Flupredniden, Prednicarbat, Desonid;
Diagnostika;
Hämostyptika/Antihämorrhagika;
Hypnotika, Sedativa; Hypophysen-, Hypothalamushormonen, regulatorischen Peptiden
und ihren Hemmstoffen;
Immuntherapeutika und Zytokinen;
Lokalanaesthetika;
Migränemittel;
Narkosemittel;
Nebenschilddrüsenhormonen, Calciumstoffwechselregulatoren;
Opthalmika;
Psychopharmaka;
Schilddrüsentherapeutika;
Sera, Immunglobulinen, Impfstoffen;
Sexualhormonen und ihren Hemmstoffen;
Zystostatika und Metastasenhemmern.

3. Verfahren nach Anspruch 1 oder 2, dadurch gekennzeichnet, daß die Teilchen der Hauptpopulation einen mittleren Durchmesser von 100 bis 500 nm und bei geeigneter Verfahrensparameter- und Hilfsstoffauswahl einen mittleren Durchmesser von 40 bis 80 nm aufweisen.

4. Verfahren nach Anspruch 1, 2 oder 3, dadurch gekennzeichnet, daß der Anteil der inneren oder Lipidphase bezogen auf die Grundrezeptur 0,1 bis 30 Gew.% und insbesondere 1 bis 10 Gew.% beträgt.

5. Verfahren nach einem der Ansprüche 1 bis 4, dadurch gekennzeichnet, daß das Teilchenmaterial Mono-, Di- und Triglyceride, Fettalkohole, deren Ester oder Ether, Wachse und Lipidpeptide sowie Mischungen derselben umfaßt.

6. Verfahren nach einem der Ansprüche 1 bis 5, dadurch gekennzeichnet, daß das Triglycerid Glycerintrilaurat, -myristat, -palmitat, -stearat und -behenat, der Fettalkohol Cetyl- und Stearylalkohol und das Wachs Cetylpalmitat sowie gebleichtes Bienenwachs umfassen.

7. Verfahren nach einem der Ansprüche 1 bis 6, dadurch gekennzeichnet, daß der Arzneistoffträger außerdem eine oder mehrere dispersionsstabilisierende Substanzen umfaßt, wobei er die dispersionsstabilisierende Substanzen bezogen auf die Grundrezeptur insbesondere in einer Menge von 0,01 bis 20 Gew.% und spezieller 0,5 bis 5 Gew.% umfaßt.

8. Verfahren nach Anspruch 7, dadurch gekennzeichnet, daß die stabilisierenden Substanzen Verbindungen aus der Reihe der Poloxamere, Poloxamine, ethoxylierten Mono- und Diglyceride, ethoxylierten Lipide und Lipoide, ethoxylierten Fettalkohole und Alkylphenole, ethoxylierten Fettsäureester, Polyglycerinether und -ester, Lecithine, Ester und Ether von Zuckern oder Zuckeralkoholen mit Fettsäuren oder Fettalkoholen, Phospholipide und Sphingolipide, Sterine, deren Ester oder Ether sowie der Mischungen dieser Verbindungen umfassen.

9. Verfahren nach Anspruch 7 oder 8, dadurch gekennzeichnet, daß die stabilisierende Substanz Eilecithin, Sojalecithin oder hydriertes Lecithin, deren Mischungen oder Mischungen aus einem oder beiden Lecithinen mit einer oder mehreren Phospholipidkomponenten, Cholesterin, Cholesterinpalmitat, Stigmasterin oder andere Sterine umfaßt.

10. Verfahren nach einem der vorhergehenden Ansprüche, dadurch gekennzeichnet, daß der Arzneistoffträger außerdem Ladungsstabilisatoren umfaßt, wobei er die Ladungsstabilisatoren bezogen auf die Grundrezeptur insbesondere in einer Menge von 0,01 bis 10 Gew.% und speziell 0,05 bis 2 Gew.% umfaßt.

11. Verfahren nach Anspruch 10, dadurch gekennzeichnet, daß die Ladungsstabilisatoren Dicetylphosphat, Phosphatidylglycerol, gesättigte oder ungesättigte Fettsäuren, Natriumcholat, Natriumglykocholat, Natriumtaurocholat oder deren Mischungen, Peptisatoren oder Aminosäuren umfassen.

12. Verfahren nach Anspruch 7, dadurch gekennzeichnet, daß der Arzneistoffträger einen oder mehrere viskositätserhöhende Stoffe umfaßt, wobei er die viskositätserhöhende Stoffe bezogen auf die Grundrezeptur insbesondere in einer Menge von 0,1 bis 10 Gew.% und speziell 0,5 bis 5 Gew.% umfaßt.

13. Verfahren nach Anspruch 12, dadurch gekennzeichnet, daß die viskositätserhöhenden Stoffe Celluloseether und -ester, Polyvinylderivate, Alginate, Polyacrylate, Xanthane und Pektine umfassen.

14. Verfahren nach Anspruch 12 oder 13, dadurch gekennzeichnet, daß der Arzneistoffträger außerdem Zucker oder Zuckeralkohole, insbesondere Glucose, Mannose, Trehalose, Mannit, Sorbit umfaßt.

15. Verfahren nach einem der Ansprüche 12 bis 14, dadurch gekennzeichnet, daß der Arzneistoffträger außerdem Ladungsträger umfaßt.

16. Verfahren nach einem der vorhergehenden Ansprüche, dadurch gekennzeichnet, daß die Teilchen in destilliertem Wasser, einer wäßrigen Lösung mit Zusätzen aus Elektrolyten, Monound Disacchariden, Polyolen oder deren Mischungen oder einer mit Wasser mischbaren Flüssigkeit dispergiert sind, wobei die Zusätze insbesondere Natriumchlorid, Mannose, Glucose, Fructose, Xylose, Trehalose, Mannit, Sorbit, Xylit und Glycerin umfassen und bezogen auf die Grundrezeptur insbesondere in einer Menge von 0,1 bis 50 Gew.% und insbesondere 1 bis 30 Gew.% vorhanden sind.

17. Verfahren nach einem der vorhergehenden Ansprüche, dadurch gekennzeichnet, daß die Teilchen lyophylisiert oder sprühgetrocknet werden.

18. Verfahren nach Anspruch 1 oder 2, dadurch gekennzeichnet, daß der Arzneistoffträger unter Ausschluß der Verwendung von halogenierten organischen Lösungsmitteln hergestellt wird.

19. Verfahren nach einem der vorhergehenden Ansprüche, dadurch gekennzeichnet, daß der Arzneistoffträger einen oder mehrere Wirkstoffe umfaßt.

20. Verfahren nach Anspruch 19, dadurch gekennzeichnet, daß der oder die Wirkstoffe in den Teilchen gelöst oder dispergiert sind oder als wäßrige Lösung in den Teilchen dispergiert sind.

21. Verfahren nach Anspruch 19, dadurch gekennzeichnet, daß der Arzneistoffträger einen oder mehrere Wirkstoffe enthält und für die intravenöse Gabe, intramuskuläre Gabe, intraartrikuläre Gabe, intracavitale Gabe, subkutane Gabe, intradermale Gabe, enterale Gabe, pulmonale Applikation sowie topische und ophtalmologische Anwendung geeignet ist.

22. Arzneistoffträger aus tensidfreien Teilchen aus Lipid, lipidähnlichem (lipoidem) Material oder Mischungen davon, die einen Durchmesser von 10 nm bis 10 µm aufweisen, durch ein Hockdruckhomogenisationsverfahren gemäß einem der Ansprüche 1 bis 20 herstellbar sind, wobei die Teilchen der Hauptpopulation einen mittleren Durchmesser zwischen 40 und 1000 nm aufweisen, und bei Raumtemperature fest sind, ausgenommen solche Träger, bei denen der Wirkstoff oder die Wirkstoffe nach einer Hochdruckdispergierung der inneren Phase in dem Dispersionsmittel in festem fein zerkleinerten Zustand nur an der Oberfläche der festen inneren Phase adsorbiert sind.

23. Verwendung einer Arzneistoffträgers gemäß Anspruch 22 zur Herstellung von Arzneimitteln, die insbesondere für die intravenöse Gabe, intramuskuläre Gabe, intraartrikuläre Gabe, intracavitale Gabe, subkutane Gabe, intradermale Gabe, enterale Gabe, pulmonale Applikation sowie topische und ophtalmologische Anwendung geeignet sind.

## Patentansprüche (Patentansprüche für folgende(n) Vertragsstaat(en): IE)

1. Verfahren zur Herstellung eines Arzneistofftrager, der tensidhaltige oder tensidfreie Teilchen aus Lipid, lipidähnlichem (lipoidem) Material oder Mischungen davon umfaßt, die einen Durchmesser von 10 nm bis 10 µm aufweisen, wobei die Teilchen der Hauptpopulation einen mittleren Durchmesser zwischen 40 und 1000 nm aufweisen und bei Raumtemperatur fest sind, dadurch gekennzeichnet, daß entweder die innere Phase (das Lipid oder Lipoid) in geschmolzenem oder erweichtem Zustand in dem Dispersionsmittel (Wasser, wäßrige Lösung oder mit Wasser mischbare Flüssigkeit) hochdruckhomogenisiert wird oder die innere Phase in festem Zustand, wobei die feste Phase fein zerklinert ist, in dem Dispersionsmittel hochdruckdispergiert wird.

2. Verfahren nach Anspruch 1, dadurch gekennzeichnet, daß die Teilchen der Hauptpopulation einen mittleren Durchmesser von 100 bis 500 nm und bog geeigneter Verfahrensparameter- und Hilfsstoffauswahl einen mittleren Durchmesser von 40 bis 80 nm aufweisen.

3. Verfahren nach Anspruch 1 oder 2, dadurch gekennzachnet, daß der Anteil der inneren oder Lipidphase bezogen auf die Grundrezeptur 0,1 bis 30 Gew.% und insbesondere 1 bis 10 Gew.% beträgt.

4. Verfahren nach einem der Ansprüche 1 bis 3, dadurch gekennzeichnet, daß das Teilchenmaterial Mono-, Di- und Triglyceride, Fettalkohole, deren Ester oder Ether, Wachse und Lipidpeptide sowie Mischungen derselben umfaßt.

5. Verfahren nach einem der Ansprüche 1 bis 4, dadurch gekennzeichnet, daß das Triglycerid Glycerintrilaurat, -myristat, -palmitat, -stearat und -behenat, der Fettalkohol Cetyl- und Stearylalkohol und das Wachs Cetylpalmitat sowie gebleichtes Bienenwachs umfassen.

6. Verfahren nach einem der Ansprüche 1 bis 5, dadurch gekennzeichnet, daß der Arzneistoffträger außerdem eine oder mehrere dispersionsstabilisierende Substanzen umfaßt, wobei er die dispersionsstabilisierende Substanzen bezogen auf die Grundrezeptur inabesondere in einer Menge von 0,01 bis 20 Gew.% und spezieller 0,5 bis 5 Gew.% umfaßt.

7. Verfahren nach Anspruch 6, dadurch gekennzeichnet, daß die stabilisierenden Substanzen Verbindungen aus der Reihe der Poloxamere, Poloxamine, ethoxylierten Mono- und Diglyceride, ethoxylierten Lipide und Lipode, ethoxylierten Fett-alkohole und Alkylphenole, ethoxylierten Fettsäureester, Polyglycerinether und -ester, Lecithine, Ester und Ether von Zukkern oder Zuckeralkoholen mit Fettsäuren oder Fettalkoholen, Phospholipide und Sphingolipide, Sterine, deren Ester oder Ether sowie der Mischungen dieser Verbindungen umfassen.

8. Verfahren nach Anspruch 6 oder 7, dadurch gekennzeichnet, daß die stabilisierende Substanz Eilecithin, Sojalecithin oder hydriertes Lecithin, deren Mischungen oder Mischungen aus einem oder beiden Lecithinen mit einer oder mehreren Phospholipdkomponenten, Cholesterin, Cholesterinpalmitat, Stigmasterin oder andere Sterine umfaßt.

9. Verfahren nach einem der vorhergehenden Ansprüche, dadurch gekennzeichnet, daß der Arzneistoffträger außerdem Ladungsstabilisatoren umfaßt, wobei er die Ladungsstabilisatoren bezogen auf die Grundrezeptur insbesondere in einer Menge von 0,01 bis 10 Gew.% und speziell 0,05 bis 2 Gew.% umfaßt.

10. Verfahren nach Anspruch 9, dadurch gekennzeichnet, daß die Ladungsstabilisatoren Dicetylphosphat, Phosphatidylglycerol, gesättigte oder ungesättigte Fettsäuren, Natriumcholat, Natriumglykocholat, Natriumtaurocholat oder deren Mischungen, Peptisatoren oder Aminosäuren umfassen.

11. Verfahren nach Anspruch 6, dadurch gekennzeichnet, daß der Arzneistoffträger einen oder mehrere viskoitätserhöhende Stoffe umfaßt, wobei er die viskositätserhöhende Stoffe bezogen auf die Grundrezeptur insbesondere in einer Menge von 0,1 bis 10 Gew.% und speziell 0,5 bis Gew.% umfaßt.

12. Verfahren nach Anspruch 11, dadurch gekennzeichnet, daß die viskositätserhöhenden Stoffe Celluloseether und - ester, Polyvinylderivate, Alginate, Polyacrylate, Xanthane und Pektine umfassen.

13. Verfahren nach Anspruch 11 oder 12, dadurch gekennzeichnet, daß der Arzneistoffträger außerdem Zucker oder Zuckeralkohole, insbesondere Glucose, Mannose, Trehalose, Mannit, Sorbit umfaßt.

14. Verfahren nach einem der Ansprüche 11 bis 13, dadurch gekennzeichnet, daß der Arzneistoffträger außerdem Ladungsträger unfaßt.

15. Verfahren nach einem der vorhergehenden Ansprüche, dadurch gekennzeichnet, daß die Teilchen in destilliertem Wasser, einer wäßrigen Lösung mit Zusätzen aus Elektrolyten, Mono- und Disacchariden, Polyolen oder deren Mischungen oder einer mit Wasser mischbaren Flüssigkeit dispergiert sind, wobei die Zusätze insbesondere Natriumchlorid, Mannose, Glucose, Fructose, Xylose, Trehalose, Mannit, Sorbit, Xylit und Glycerin umfassen und bezogen auf die Grundrezeptur insbesondere in einer Menge von 0,1 bis 50 Gew.% und insbesondere 1 bis 30 Gew.% vorhanden sind.

16. Verfahren nach einem der vorhergehenden Ansprüche, dadurch gekennzeichnet, daß die Teilchen lyophylisiert oder sprühgetrocknet werden.

17. Verfahren nach Anspruch 1, dadurch gekennzeichnet, daß der Arzneistoffträger unter Ausschluß der Verwendung von halogenierten organischen Lösungsmitteln hergestellt wird.

18. Verfahren nach einem der vorhergehenden Ansprüche, dadurch gekennzeichnet, daR der Arzneistoffträger keinen, einen oder mehrere Wirkstoffe umfaßt.

19. Verfahren nach Anspruch 18, dadurch gekennzeichnet, daß der oder die Wirkstoffe in den Teilchen gelöst oder dispergiert sind, an deren Oberfläche adsorbiert and oder als wäßrige Lösung in den Teilchen dispergiert sind.

20. Verfahren nach Anspruch 18, dadurch gekennzeichnet, daß der Arzneistoffträger einen oder mehrere Wirkstoffe enthält und für die intravenöse Gabe, intramuskoläre Gabe, intraartrikuläre Gabe, intracavitale Gabe, subkutane Gabe, intradermale Gabe, enterale Gabe, pulmonale Applikation sowie topische und ophtalmologische Anwendung geeignet ist.

21. Arzneistoffträger aus tensidfreien Teilchen aus Lipid, lipidähnlichem (lipoidem) Material oder Mischungen davon, die einen Durchmesser von 10 nm bis 10 µm aufweisen, durch ein Hochdruokhomogenisationsverfahren gemäß einem der Ansprüche 1 bis 19 herstellbar sind, wobei die Teilchen der Hauptpopulation einen mittleren Durchmesser zwischen 40 und 1000 nm aufweisen, und bei Raumtemperatur fest sind.

22. Verwendung einer Arzneistoffträgers gemäß Anspruch 21 zur Herstellung von Arzneimitteln, die insbesondere für die intravenöse Gabe, intramuskuläre Gabe, intraartrikoläre Gabe, intracavitale Gabe, subkutane Gabe, intradermale Gabe, enterale Gabe, pulmonale Applikation sowie topische und ophtalmologische Anwendung geeignet sind.

## Claims (Claims for the following Contracting State(s): AT, BE, CH, DE, DK, ES, FR, GB, GR, IT, LI, LU, MC, NL, SE)

1. Process for the manufacture of a drug carrier which comprises tensidecontaining or tenside-free particles of lipid or lipid-like (lipoid) material, or mixtures thereof, which have a diameter of 10 nm to 10 µm, whereby the particles of the main population have an average diameter of between 40 and 1000 nm and are solid at room temperature, characterised by the fact that either the inner phase (the lipid or lipoid) is homogenised under high pressure in the dispersion medium (water, aqueous solution or a liquid which can be mixed with water) in a melted or softened state, whereby, where melted lipid or lipoid is used as the inner phase during homogenisation under high pressure, one or more active substances are also present and are encapsulated in the lipid or lipoid, or the inner phase is dispersed under high pressure in the dispersion medium in a solid state, whereby the solid phase is finely broken down.

2. Process for the manufacture of a drug carrier which comprises tensidecontaining or tenside-free particles of lipid or lipid-like (lipoid) material, or mixtures thereof, which have a diameter of 10 nm to 10 µm, whereby the particles of the main population have an average diameter of between 40 and 1000 nm and are solid at room temperature, characterised by the fact that either the inner phase (the lipid or lipoid) is homogenised under high pressure in the dispersion medium (water, aqueous solution or a liquid which can be mixed with water) in a melted or softened state, or the inner phase is dispersed under high pressure in the dispersion medium in a solid state, whereby the solid phase is finely broken down, and the drug carrier includes one or more active substances which are selected from:
Analgesics/antirheumatics selected from
morphine, codeine, piritamide, fentanyl and fentanyl derivatives, levomethadone, tramadol, diclofenac, ibuprofen, naproxen, piroxicam, penicillamine;
Antiallergics;
Antibiotics/chemotherapeutics selected from
polypeptide antibiotics; anti-malaria drugs, the virunstatics ganciclovir, foscarnet, zidovudine, aciclovir and dapsone, fosfomycin, fusafungine, trimethoprim;
Antiepileptics;
Antimycotlcs selected from
nystatin, natamycin, amphotericin B, flucytosine, miconazole, fluconazole, itraconazole, clotrimazole, econazole, tioconazole, fenticonazole, bifonasole, ketoconzole, isoconazole, tolnaftate;
Corticosteroids selected from
aldosterone, fludrocortisone, betamethasone, dexamethasone, fluocortolone, prednisolone, prednylidene, cloprednol, methylprednisolone;
Dermatological drugs selected from
a) the antibiotics tetracycline, erythromycin, neomycin, gentamycin, clindamycin, framycetin, tyrothricin, chlorotetracycline, mipirocin, fusidinic acid
b) the virustatics podophyllotoxin, vidarabine, tromantadine,
c) the corticosteroids amcinodid, fluprednidene, alclometasone, clobetasol, diflorasone, halcinonid, fluocinolone, clocortolone, flumetasone, diflucortolone, fludroxycortid, halomethasone, desoxymethasone, fluocinolide, fluocortinbutyl, fluprednidene, prednicarbate, desonid;
Diagnostics;
Haemostyptics/antihaemorragics;
Hypnotics, sedatives; Hypophysis and hypothalmus hormones, regulatory peptides and their
inhibitors;
Immune therapeutics and cytokines;
Local anaesthetics;
Migraine treatments;
Narcotics;
Parathyroid hormones, calcium metabolism regulators;
Opthalmics;
Psychopharmaceutics;
Thyroid drugs;
Serums, immunoglobulins, vaccines;
Sexual hormones and their inhibitors;
Cystostatics and metastasis inhibitors.

3. Process as in claim 1 or 2, characterised by the fact that the particles of the main population have an average diameter of 100 to 500 nm and, with appropriate selection of process parameters and auxiliary media, have an average diameter of 40 to 80 nm.

4. Process as in claim 1, 2 or 3, characterised by the fact that the proportion of the inner or lipid phase in relation to the basic preparation is 0.1 to 30 % by weight and, especially, 1 to 10 % by weight.

5. Process as in one of the claims 1 to 4, characterised by the fact that the particle material comprises monoglyceride, diglyceride and triglyceride, fatty alcohols and the esters or ethers thereof, waxes and lipid peptides or mixtures of these.

6. Process as in one of the claims 1 to 5, characterised by the fact that the triglyceride comprises glycerine trilaurate, glycerine myristate, glycerine palmitate, glycerine stearate and glycerine behenate, that the fatty alcohol comprises cetyl and stearyl alcohol and the wax comprises cetyl palmitate and bleached beeswax.

7. Process as in one of the claims 1 to 6, characterised by the fact that, in addition, the drug carrier includes one or more dispersionstabilising substances, whereby the dispersion-stabilising substances are included in a quantity of 0.01 to 20 % by weight in relation to the basic preparation, ideally 0.5 to 5 % by weight.

8. Process as in claim 7, characterised by the fact that the stabilising substances comprise compounds from the series of poloxamers, poloxamins, ethoxylated monoglycerides and diglycerides, ethoxylated lipids and lipoids, ethoxylated fatty alcohols and alkyl phenols, ethoxylated fatty acid esters, polyglycerine ethers and esters, lecithins, esters and ethers of sugars or sugar alcohols with fatty acids and fatty alcohols, phospholipids and sphingolipids, sterols or the esters and ethers thereof, as well as mixtures of these compounds.

9. Process as in claim 7 or 8, characterised by the fact that the stabilising substance comprises egg-lecithin, soya-lecithin or hydrogenated lecithin, mixtures thereof, or mixtures of one or both lecithins with one or more phospholipid components, cholesterin, cholesterin palmitate, stigmaterin or other sterols.

10. Process as in one of the preceding claims, characterised by the fact that, in addition, the drug carrier includes load stabilisers in a quantity of 0.1 to 10 % by weight and especially 0.05 to 2 % by weight in relation to the basic preparation.

11. Process as in claim 10, characterised by the fact that the load stabilisers comprise dicetyl phosphate, phosphatidylglycerol, saturated or unsaturated fatty acids, sodium cholate, sodium glycocholate, sodium taurocholate or mixtures thereof, peptisators or amino acids.

12. Process as in claim 7, characterised by the fact that the drug carrier comprises one or more viscosity-increasing substances, whereby the viscosity-increasing substances are included in a quantity of 0.1 to 10 % by weight in particular, ideally 0.5 to 5 % by weight, in relation to the basic preparation.

13. Process as in claim 12, characterised by the fact that the viscosityincreasing substances comprise cellulose ethers and esters, polyvlnyl derivatives, alginates, polyacrylates, xanthanes and pectins.

14. Process as in claim 12 or 13, characterised by the fact that the drug carrier also comprises sugar or sugar alcohols, especially glucose, mannose, trehalose, mannitol and sorbitol.

15. Process as in one of the claims 12 to 14, characterised by the fact that the drug carrier also comprises load carriers.

16. Process as in one of the preceding claims, characterised by the fact that the particles are dispersed in distilled water, in an aqueous solution with additives of electrolytes, monosaccharides and disaccharides, polyols or mixtures thereof or a liquid that can be mixed with water, whereby the additives comprise, in particular, sodium chloride, mannose, glucose, fructose, xylose, trehalose, mannitol, sorbitol, xylitol and glycerol, preferably in a quantity of 0.1 to 50 % by weight and especially 1 to 30 % by weight in relation to the basic preparation.

17. Process as in one of the preceding claims, characterised by the fact that the particles are lyophilised or spray-dried.

18. Process as in claim 1 or 2, characterised by the fact that the drug carrier is manufactured without the use of halogenated organic solvents.

19. Process as in one of the preceding claims, characterised by the fact that the drug carrier includes one or more active substances.

20. Process as in claim 19, characterised by the fact that the active substance or substances are dissolved or dispersed in the particles or dispersed in the particles as an aqueous solution.

21. Process as in claim 19, characterised by the fact that the drug carrier contains one or more active substances and is suitable for intravenous administration, intramuscular administration, intra-arthricular administration, intracavital administration, subcutaneous administration, intradermal administration, enteral administration, pulmonary application and topical and opthalmological application.

22. Drug carrier consisting of tenside-free particles of lipid or lipidlike (lipoid) material, or mixtures thereof, with a diameter of 10 nm to 10 µm, which can be manufactured by means of a high-pressure homogenisation process in accordance with one of the claims 1 to 20, whereby the particles of the main population have an average diameter of between 40 and 1000 nm and are solid at room temperature, excluding such carriers where the active substance or substances are only adsorbed onto the surface of the solid inner phase following dispersion under high pressure of the inner phase, in a finely broken-down state, in the dispersion medium.

23. Use of a drug carrier as in claim 22 for the manufacture of drugs which are, in particular, suitable for intravenous administration, intramuscular administration, intra-arthricular administration, intracavital administration, subcutaneous administration, intradermal administration, enteral administration, pulmonary application and topical and opthalmological application.

## Claims (Claims for the following Contracting State(s): IE)

1. Process for the manufacture of a drug carrier which comprises tenside-containing or tensid-free particles of lipid or lipid-like (lipoid) material, or mixtures thereof, which have a diameter of 10 nm to 10 µm, whereby the particles of the main population have an average diameter of between 40 and 1000 nm and are solid at room temperature, characterised by the fact that either the inner phase (the lipid or lipoid), is homogenised under high pressure in the dispersion medium (water, aqueous solution or a liquid which can be mixed with water) in a melted or softened state, or the inner phase is dispersed under high pressure in the dispersion medium in a solid state, whereby the solid phase is finely broken down.

2. Process as in claim 1, characterised by the fact that the particles of the main population have an averaged diameter of 100 to 500 nm and, with appropriate selection of process parameters and auxiliary media, have an average diameter of 40 to 80 nm.

3. Process as in claim 1 or 2, characterised by the fact that the proportion of the inner or lipid phase in relation to the basic preparation is 0.1 to 30 % by weight and, especially, 1 to 10 % by weight.

4. Process as in one of the claims 1 to 3, characterised by the fact that the particle material comprises monoglyceride, diglyceride. triglyceride, fatty alcohols and the esters or ethers thereof, waxes and lipid peptides or mixtures of these.

5. Process as in one of the claims 1 to 4, characterised by the fact that the triglyceride comprises glycerine trilaurate, glycerine myristate, glycerine palmitate, glycerine stearate and glycerine behenate, that the fatty alcohol comprises cetyl and stearyl alcohol and the wax comprises cetyl palmitate and bleached beeswax.

6. Process as in one of the claims 1 to 5, characterised by the fact that, in addition, the drug carrier includes one or more dispersion-stabilising substances, whereby the dispersion-stabilising substances are included in a quantity of 0.1 to 20 % by weight in relation to the basic preparation, ideally 0.5 to 5 % by weight.

7. Process as in claim 6, characterised by the fact that the stabilising substances comprise compounds from the series of poloxamers, poloxamins, ethorylated monoglycerides and diglycerides, ethorylated lipids and lipoids, ethoxylated fatty alcohols and alkyl phenols, ethoxylated fatty acid esters, polyglycerine ethers and esters, lecithins, esters and ethers of sugars or sugar alcohols with fatty acids or fatty alcohols, phospholipids and sphingolipids, sterols or the esters and ethers thereof, as well as mixtures of these compounds.

8. Process as in claim 6 or 7, characterised by the fact that the stabilising substance comprises egg-lecithin, soya-lecithin or hydrogenated lecithin, mixtures thereof, or mixtures of one or both lecithins with one or more phospholipid components, cholesterin, cholesterin palmitate, stigmaterin or other sterols.

9. Process as in one of the preceding claims, characterised by the fact that, in addition, the drug carrier includes load stabilisers in a quantity of 0.01 to 10 % by weight and especially 0.05 to 2 % by weight.

10. Process as in claim 9, characterised by the fact that the load stabilisers comprise dicetyl phosphate, phosphatidylglycerol saturated or unsaturated fatty acids, sodium cholate, sodium glycocholate, sodium taurocholate or mixtures thereof, peptisators or amino acids.

11. Process as in claim 6, characterised by the fact that the drug carrier comprises one or more viscosity-increasing substances, whereby the viscosity-increasing substances are included in a quantity' of 0.1 to 10 % by weight, ideally 0.5 to 5 % by weight in relation to the basic preparation.

12. Process as in claim 11, characterised by the fact that the viscosity-increasing substances comprise cellulose ethers and esters, polyvinyl derivatives, alginates, polyacrylates, xanthanes and pectins.

13. Process as in claim 11 or 12, characterised by the fact that the drug carrier also comprises sugar or sugar alcohols, especially glucose, mannose, trehalose, mannitol and sorbitol.

14. Process as in one of the claims 11 to 13, characterised by the fact that the drug carrier also comprises load carriers.

15. Process as in one of the preceding claims, characterised by the fact that the particles are dispersed in distilled water, in an aqueous solution with additives of electrolytes, monosaccharides and disaccharides, polyols or mixtures thereof or a liquid that can be mixed with water, whereby the additives comprise, in particular, sodium chloride, mannose, glucose, fructose, xylose, trehalose, mannitol, sorbitol, xylitol and glycerol, preferably in a quantity of 0.1 to 50 % by weight and especially 1 to 30 % by weight in relation to the basic preparation.

16. Process as in one of the preceding claims, characterised by the fact that the particles are lyophilised or spray-dried.

17. Process as in claim 1, characterised by the fact that the drug carrier is manufactured without the use of halogenated organic solvents.

18. Process as in one of the preceding claims, characterised by the fact that the drug carrier includes no active substance, or one or more active substances.

19. Process as in claim 18, characterised by the fact that the active substance or substances are dissolved or dispersed in the particles, adsorbed on the surface of the particles or dispersed in the particles as an aqueous solution.

20. Process as in claim 18, characterised by the fact that the drug carrier contains one or more active substances and is suitable for intravenous administration, intramuscular administration, intra-arthricular administration, intracavital administration, subcutaneous administration, intradermal administration, enteral administration, pulmonary application and topical and opthalmological application.

21. Drug carrier consisting of tensid-free particles of lipid or lipid-like (lipoid) material, or mixtures thereof, with a diameter of 10 nm to 10 nµ, which can be manufactured by means of a high-pressure homogenisation process in accordance with one of the claims 1 to 19, whereby the particles of the main population have an average diameter of between 40 and 1000 nm and are solid at room temperature.

22. Use of a drug carrier as in claim 21 for the manufacture of drugs which are, in particular, suitable for intravenous administration, intramuscular administration, intra-arthricular administration, intracavital administration, subcutaneous administration, intradermal administration, enteral administration, pulmonary application and topical and opthalmological application.

## Revendications (Revendications pour l'(les) Etat(s) contractant(s) suivant(s): AT, BE, CH, DE, DK, ES, FR, GB, GR, IT, LI, LU, MC, NL, SE)

1. Procédé pour la préparation d'un véhicule de substance médicamenteuse, qui comprend des particules contenant un tensioactif ou exemptes de tensioactif, à base de lipide, d'une substance de type lipidique (lipoïdique) ou de mélanges de ceux-ci, qui présentent un diamètre de 10 nm à 10 µm, la majeure partie des particules ayant un diamètre moyen compris entre 10 et 1 000 nm, et étant solides à la température ambiante, caractérisé en ce que soit la phase interne (le lipide ou lipoïde) est homogénéisée sous haute pression, à l'état fondu ou ramolli, dans le milieu de mise en dispersion (eau, solution aqueuse ou liquide miscible à l'eau) étant entendu que lors de l'utilisation du lipide ou lipoïde fondu en tant que phase interne pendant l'homogénéisation sous haute pression, une ou plusieurs substances actives sont également présentes et incorporées au lipide ou lipoïde, soit la phase interne est dispersée sous haute pression, à l'état solide, la phase solide étant finement divisée, dans le milieu de mise en dispersion.

2. Procédé pour la préparation d'un véhicule de substance médicamenteuse, qui comprend des particules contenant un tensioactif ou exemptes de tensioactif, à base de lipide, d'une substance de type lipidique (lipoïdique) ou de mélanges de ceux-ci, qui présentent un diamètre de 10 nm à 10 µm, la majeure partie des particules ayant un diamètre moyen compris entre 40 et 1 000 nm, et étant solides à la température ambiante, caractérisé en ce que soit la phase interne (le lipide ou lipoïde) est homogénéisée sous haute pression, à l'état fondu ou ramolli, dans le milieu de mise en dispersion (eau, solution aqueuse ou liquide miscible à l'eau), soit la phase interne est dispersée sous haute pression, à l'état solide, la phase solide étant finement divisée, dans le milieu de mise en dispersion, et le véhicule de substance médicamenteuse comprend une ou plusieurs substances actives, qui sont choisies parmi :
Analgésiques/antirhumatismaux choisis parmi :
morphine, codéine, piritamide, fentanyle et dérivés de fentanyle, lévométhadone, tramadole, diclofénac, ibuprofène, naproxène, piroxicam, pénicillamine;
Antiallergiques;
Antibiotiques/agents chimiothérapeutiques choisis parmi:
antibiotiques polypeptidiques, agents antipaludéens, l'antiviral ganciclovir, le foscarnet, la zidovudine, l'aciclovir et la dapsone, la fosfomycine, la fusafungine, le triméthoprime;
Antiépileptiques;
Antifongiques choisis parmi :
nystatine, natamycine, amphotéricine B, flucytosine, miconazole, fluconazole, itraxonazole, clotrimazole, éconazole, tioconazole, fenticonazole, bifonazole, kétoconazole, isoconazole, tolnaftate;
Corticoïdes choisis parmi :
aldostérone, fluodrocortisone, bétaméthasone, dexaméthasone, fluocortolone, prednisolone, prednylidène, cloprednole, méthylprednisolone.
Produits dermatologiques choisis parmi :
a) les antibiotiques tétracycline, érythromycine, néomycine, gantamycine, clindamycine, framycétine, tyrothricine, chlorotétracycline, mipirocine, acide fusidique;
b) les antiviraux podophyllotoxine, vidarabine, tromantadine;
c) les corticoïdes amcinonide, fluprednidène, alclométhasone, clobétasole, diflorasone, halcinonide, fluocinolone, clocortolone, fluméthasone, difluocortolone, fludroxycortide, halométhasone, désoximéthasone, fluocinolide, fluocortinbutyle, fluprednidène, prednicarbate, désonide;
Produits diagnostiques;
Hémostatiques/antihémorragiques;
Hypnotiques, sédatifs;
Hormones hypophysaires et hypothalamiques, peptides régulateurs et leurs inhibiteurs;
Agents immunothérapeutiques et cytokines;
Anesthésiques locaux;
Antimigraineux;
Narcotiques;
Hormones parathyroïdiennes, régulateurs du métabolisme du calcium;
Produits ophtalmiques;
Neuroleptiques;.
Agents thyroïdiens;
Sérums, immunoglobulines, vaccins;
Hormones sexuelles et leurs inhibiteurs.

3. Procédé selon la revendication 1 ou 2, caractérisé en ce que les particules ont en majorité un diamètre moyen de 100 à 500 nm et, dans le cas d'un choix d'adjuvant et de paramètres de procédé appropriés, un diamètre moyen de 40 à 80 nm.

4. Procédé selon la revendication 1 ou 2, caractérisé en ce que la proportion de la phase lipidique ou interne, par rapport à la composition de base, va de 0,1 à 30% en poids et en particulier de 1 à 10% en poids.

5. Procédé selon l'une des revendications 1 à 4, caractérisé en ce que le matériau des particules comprend des mono-, di et triglycérides, des alcools gras, leurs esters ou éthers, des cires et des lipopeptides, ainsi que des mélanges de ceux-ci.

6. Procédé selon l'une des revendications 1 à 5, caractérisé en ce que le triglycéride comprend le trilaurate, -myristate, -palmitate, -stéarate et -béhénate de glycérol, l'alcool gras comprend l'alcool cétylique et l'alcool stéarylique, et la cire comprend le palmitate de cétyle ainsi que la cire blanche d'abeille.

7. Procédé selon l'une des revendications 1 à 6, caractérisé en ce que le véhicule de substance médicamenteuse comprend en outre une ou plusieurs substances stabilisant la dispersion, celui-ci comprenant les substances stabilisant la dispersion, par rapport à la composition de base, en particulier en une proportion de 0,01 à 20% en poids, et plus particulièrement de 0,5 à 5% en poids.

8. Procédé selon la revendication 7, caractérisé en ce que les substances stabilisantes comprennent des composés choisis parmi des Poloxamères, Poloxamines, mono- et diglycérides éthoxylés, lipides et lipoïdes éthoxylés alcools gras et alkylphénols éthoxylés, esters d'acides gras éthoxylés, polyéthers et -esters de glycérol, lécithines, esters et éthers de sucres ou de sucre-alcools avec des acides gras ou des alcools gras, phospholipides et sphingolipides, stérols, leurs esters ou éthers ainsi que des mélanges de ces composes.

9. Procédé selon la revendication 7 ou 8, caractérisé en ce que la substance stabilisante comprend la lécithine d'oeuf, la lécithine de soja ou la lécithine hydrogénée, leurs mélanges ou des mélanges d'une lécithine ou des deux avec un ou plusieurs composants phospholipidiques, le cholestérol, le palmitate de cholestérol, le stigmastérol ou d'autres stérols.

10. Procédé selon l'une des revendications précédentes, caractérisé en ce que le véhicule de substance médicamenteuse comprend en outre des stabilisants de charge, celui-ci comprenant les stabilisants de charge, par rapport à la composition de base, en particulier en une proportion de 0,01 à 10% en poids et plus particulièrement de 0,05 à 2% en poids.

11. Procédé selon la revendication 10, caractérisé en ce que les stabilisants de charge comprennent le phosphate de dicétyle, le phosphatidyglycérol, des acides gras saturés ou insaturés, le cholate de sodium, le glycocholate de sodium, le taurocholate de sodium ou des mélanges de ceux-ci, des peptisateurs ou des aminoacides.

12. Procédé selon la revendication 7, caractérisé en ce que le véhicule de substance médicamenteuse comprend une ou plusieurs substances augmentant la viscosité, celui-ci comprenant les substances augmentant la viscosité, par rapport à la composition de base, en particulier en une proportion de 0,1 à 10% en poids et plus particulièrement de 0,5 à 5% en poids.

13. Procédé selon la revendication 12, caractérisé en ce que les substances augmentant la viscosité comprennent des éthers et esters de cellulose, des dérivés polyvinyliques, des alginates, des polyacrylates, des xanthannes et des pectines.

14. Procédé selon la revendication 12 ou 13, caractérisé en ce que le véhicule de substance médicamenteuse comprend en outre des sucres ou des sucrealcools, en particulier le glucose, le mannose, le tréhalose, le mannitol, le sorbitol.

15. Procédé selon l'une des revendications 12 à 14, caractérisé en ce que le véhicule de substance médicamenteuse comprend en outre des porteurs de charge.

16. Procédé selon l'une des revendications précédentes, caractérisé en ce que les particules sont dispersées dans de l'eau distillée, une solution aqueuse avec addition d'électrolytes, de mono- et disaccharides, de polyols ou des mélanges de ceux-ci, ou dans un liquide miscible à l'eau, les additifs comprenant en particulier le chlorure de sodium, le mannose, le glucose, le fructose, le xylose, le tréhalose, le mannitol, le sorbitol, le xylitol et le glycérol, et étant présents, par rapport à la composition de base, en particulier en une proportion de 0,1 à 50% en en poids et plus particulièrement de 1 à 30% en poids.

17. Procédé selon l'une des revendications précédentes, caractérisé en ce que les particules sont lyophilisées ou séchées par atomisation.

18. Procédé selon la revendication 1 ou 2, caractérisé en ce que le véhicule de substance médicamenteuse est préparé avec exclusion de l'utilisation de solvants organiques halogénés.

19. Procédé selon l'une des revendications précédentes, caractérisé en ce que le véhicule de substance médicamenteuse comprend une ou plusieurs substances actives.

20. Procédé selon la revendication 19, caractérisé en ce que la ou les substances actives est (sont) dissoute(s) ou dispersée(s) dans les particules, est (sont) adsorbée(s) sur leur surface ou est (sont) dispersée(s) sous forme de solution aqueuse dans les particules.

21. Procédé selon la revendication 19, caractérisé en ce que le véhicule de substance médicamenteuse contient une ou plusieurs substances actives et est approprié à l'administration intraveineuse, l'administration intramusculaire, l'administation intra-articulaire, l'administration intracavitaire, l'administration sous-cutanée, l'administration intradermique, l'administration entérale, l'administration pulmonaire ainsi que l'application locale et l'application ophtalmologique.

22. Véhicule de substance médicamenteuse, constitué de particules, exemptes de tensioactif, à base d'un lipide, d'une substance de type lipide (lipoïde) ou de mélanges de ceux-ci, qui présentent un diamètre de 10 nm à 10 µmn peuvent être obtenues par un procédé d'homogénéisation à haute pression selon l'une des revendications 1 à 20, la majorité des particules présentant un diamètre moyen compris entre 40 et 1 000 nm et étant solides à la température ambiante, les véhicules dans lesquels le ou les substances actives sont seulement adsorbées à la surface de la phase interne solide après mise en dispersion sous haute pression de la phase interne dans le milieu de dispersion, à l'état solide finement divisé, étant exclus.

23. Utilisation d'un véhicule de substance médicamenteuse selon la revendication 22, pour la fabrication de médicaments qui sont appropriés à l'utilisation en particulier pour l'administration intraveineuse, l'administration intramusculaire, l'administration intra-articulaire, l'administration intracavitaire, l'administration sous-cutanée, l'administration intradermique, l'administration entérale, l'administration pulmonaire ainsi que l'application locale et l'application ophtalmologique et l'application opthalmologique.

## Revendications (Revendications pour l'(les) Etat(s) contractant(s) suivant(s): IE)

1. Procédé pour la préparation d'un véhicule de substance médicamenteuse, qui comprend des particules contenant un tensioactif ou exemptes de tensioactif, à base de lipide, d'une substance de type lipidique (lipoïdique) ou de mélanges de ceux-ci, qui présentent un diamètre de 10 nm à 10 µm, la majeure partie des particules ayant un diamètre moyen compris entre 40 et 1 000 nm, et étant solides à la température ambiante, caractérisé en ce que soit la phase interne (le lipide ou lipoïde) est homogénéisée sous haute pression, à l'état fondu ou ramolli, dans le milieu de mise en dispersion (eau, solution aqueuse ou liquide miscible à l'eau), soit la phase interne est dispersée sous haute pression, à l'état solide, la phase solide étant finement divisée, dans le milieu de mise en dispersion.

2. Procédé selon la revendication 1, caractérisé en ce que les particules ont en majorité un diamètre moyen de 100 à 500 nm et, dans le cas d'un choix d'adjuvant et de paramètres de procédé appropriés, un diamètre moyen de 40 à 80 nm.

3. Procédé selon la revendication 1 ou 2, caractérisé en ce que la proportion de la phase lipidique ou interne, par rapport à la composition de base, va de 0,1 à 30 % en poids et en particulier de 1 à 10 % en poids.

4. Procédé selon l'une des revendications 1 à 3, caractérisé en ce que le matériau des particules comprend des mono-, di- et triglycérides, des alcools gras, leurs esters ou éthers, des cires et des lipopeptides, ainsi que des mélanges de ceux-ci.

5. Procédé selon l'une des revendications 1 à 4, caractérisé en ce que le triglycéride comprend le trilaurate, -myristate, -palmitate, - stéarate et -béhénate de glycérol, l'alcool gras comprend l'alcool cétylique et l'aicool stéarylique₁ et la cire comprend le palmitate de cétyle ainsi que la cire blanche d'abeille.

6. Procédé selon l'une des revendications 1 à 5, caractérisé en ce que le véhicule de substance médicamenteuse comprend en outre une ou plusieurs substances stabilisant la dispersion, celui-ci comprenant les substances stabilisant la dispersion, par rapport à la composition de base, en particulier en une proportion de 0,01 à 20 % en poids, et plus particulièrement de 0,5 à 5 % en poids.

7. Procédé selon la revendication 6 caractérisé en ce que les substances stabilisantes comprennent des composés choisis parmi des Poloxamères, Poloxamines, mono- et diglycérides éthoxylés, lipides et lipoïdes éthoxylés alcools gras et alkylphénols éthoxylés, esters d'acides gras éthoxylés, polyéters et -esters de glycérol, lécithines, esters et éthers de sucres ou de sucre-alcools avec des acides gras ou des alcools gras, phospholipides et sphingolipides, stérols, leurs esters ou éthers ainsi que des mélanges de ces composés.

8. Procédé selon la revendication 6 ou 7, caractérisé en ce que la substance stabilisante comprend la lécithine d'oeuf, la lécithine de soja ou la lécithine hydrogénée, leurs mélanges ou des mélanges d'une lécithine ou des deux avec un ou plusieurs composants phospholipidiques, le cholestérol, le palmitate de cholestérol, le stigmastérol ou d'autres stérols.

9. Procédé selon l'une des revendications précédentes, caractérisé en ce que le véhicule de substance médicamenteuse comprend en outre des stabilisants de charge, celui-ci compreflant les stabilisants de charge, par rapport à la composition de base, en particulier en une proportion de 0,01 à 10 % en poids et plus particulièrement de 0,05 à 2 % en poids.

10. Procédé selon la revendication 9, caractérisé en ce que les stabilisants de charge comprennent le phosphate de dicétyle, le phosphatidylglycérol, des acides gras saturés ou insaturés, le cholate de sodium, le glycocholate de sodium, le taurocholate de sodium ou des mélanges de ceuxci, des peptisateurs ou des aminoacides.

11. Procédé selon la revendication 6, caractérisé en ce que le véhicule de substance médicamenteuse comprend une ou plusieurs substances augmentant la viscosité, celui-ci comprenant les substances augmentant la viscosité, par rapport à la composition de base, en particulier en une proportion de 0,1 à 10 % en poids et plus particulièrement de 0,5 à 5 % en poids.

12. Procédé selon la revendication 11, caractérisé en ce que les substances augmentant la viscosité comprennent des éthers et esters de cellulose, des dérivés polyvinyliques, des alginates. des polyacrylates. des xanthannes et des peccines.

13. Procédé selon la revendication 11 ou 12, caractérisé en ce que le véhicule de substance médicateenteuse comprend en outre des sucres ou sucre-alcools , en particulier le glucose, le mannose, le tréhalose, le mannitol, le sorbitol.

14. Procedé selon l'une des revendications 11 à 13, caractérisé en ce que le véhicule de substance médicamenteuse comprend en outre des porteurs de charge.

15. Procedé selon l'une des revendications précédentes, caractérisé en ce que les particules sont dispersées dans de l'eau distillée, une solution aqueuse avec addition d'électrolytes, de mono- et disaccharides, de polyols ou des mélanges de ceux-ci, ou dans un liquide miscible à l'eau, les additifs comprenant en particulier le chlorure de sodium, le mannose, le glucose, le fructose, le xylose, le tréhalose, le mannitol, le sorbitol, le xylitol et le glycéroi, et étant présents, par rapport à la composition de base, en particulier en une proportion de 0,1 à 50 % en poids et plus particulièrement de 1 à 30 % en poids.

16. Procedé selon l'une des revendications précédentes, caractérisé en ce que les particules sont lyophilisées ou séchées par atomisation.

17. Procédé selon la revendication 1, caractérisé en ce que le véhicule de substance médicamenteuse est préparé avec exclusion de l'utilisation de solvants organiques halogènés.

18. Procédé selon l'une des revendications précédentes, caractérisé en ce que le véhicule de substance médicamenteuse comprend une ou plusieurs substances actives ou n'en comprend aucune.

19. Procédé selon la revendication 18, caractérisé en ce que la ou les substances actives est(sont) dissoute(s) ou dispersée(s) dans les particules, est(sont) adsorbée(s) sur leur surface ou est (sont) dispersée(s) sous forme de solution aqueuse dans les particules.

20. Procédé selon la revendication 18, caractérisé en ce que le véhicule de substance médicamenteuse contient une ou plusieurs substances actives et est approprié à l'administration intraveineuse, l'administration intramusculaire, l'administration intra-articulaire, l'administration intracavitaire, l'administration sous-cutanée, l'administration intradermique, l'administration entérale, l'administration pulmonaire ainsi que l'application locale et l'application ophtalmologique.

21. Véhicule de substance médicamenteuse, constitué de particules, exemptes de tensioactif, à base d'un lipide, d'une substance de type lipide (lipoïde) ou de mélanges de ceux-ci, qui présentent un diamètre de 10 nm à 10 µm, peuvent être obtenues par un procédé d'homogénéisation à haute pression selon l'une des revendications 1 à 19, la majorité des particules présentant un diamètre moyen compris entre 40 et 1 000 nm et étant solides à la température ambiante.

22. Utilisation d'un véhicule de substance médicamenteuse selon la revendication 21, pour la fabrication de médicaments qui sont appropriés à l'utilisation en particulier pour l'administration intraveineuse, l'administration intramusculaire, l'administration intra-articulaire, l'administration intracavitalre, l'administration sous-cutanèe, l'administration intradermique, l'administration entérale, l'administration pulmonaire ainsi que l'application locale et l'application ophtalmologique.
